# EUROPÄISCHE PATENTSCHRIFT

(11) **EP 0 769 255 B1**
(45) Veröffentlichungstag und Bekanntmachung des Hinweises auf die Patenterteilung: **31.05.2000**
(21) Anmeldenummer: 96250227.4
(22) Anmeldetag: 11.10.1996
(51) Int. Cl.: A41C 1/10, A61F 5/03, A61F 13/14

(54) **Sich der Körperform anpassender Leibgurt**
Bodyconforming waistbelt
Ceinture réglable à la forme du corps

(30) Priorität: 19.10.1995 DE 19540234
(43) Veröffentlichungstag der Anmeldung: 23.04.1997
(73) Patentinhaber: F+E Gesellschaft für Bekleidungsinnovation mbH & Co. KG, 83098 Brannenburg (DE)
(72) Erfinder: Weber-Unger, Georg, 6330 Kufstein (AT)
(74) Vertreter: Böning, Manfred, Prof.Dr.-Ing.

(56) Entgegenhaltungen:
- DE-C- 223 248
- DE-C- 235 079
- US-A- 2 018 981
- US-A- 3 752 163
- US-A- 4 396 013
- US-A- 4 596 253
- US-A- 5 060 639

## Beschreibung

Die Erfindung betrifft einen Leibgurt, der zwei sich im Bauchbereich mehr oder weniger schuppenartig überlappende elastische Stützgurte aufweist.

Ein Leibgurt der vorstehenden Art ist aus der DE-A-223 248 bekannt. Bei dem bekannten Leibgurt deckt der obere Stützgurt praktisch den gesamten Bauchbereich ab, während der untere Stützgurt sich vom Unterbauch bis in den Leistenbereich erstreckt. Beide Stützgurte überlappen sich ständig auf einem großen Teil ihrer beträchtlichen Höhe. Ziel der Überlappung ist es, im überlappten Bereich eine besonders große Stützwirkung zu erzielen.

Aus der US-A-3 752 163 ist außerdem eine Leibbinde bekannt, die aus drei über Längsnähte miteinander verbundenen Abschnitten besteht, von denen zwei drei durch Quernähte miteinander verbundene Unterabschnitte aufweisen. Bei dieser Leibbinde ermöglichen die Quernähte eine geringfügige Überlappung der Unterabschnitte.

Sowohl der bekannte Leibgurt als auch die bekannte Leibbinde vermögen insofern nicht voll zu befriedigen als sie aufgrund ihrer Quersteifigkeit in der Sitzposition der sie tragenden Personen insbesondere zu störenden Druckbelastungen in den Leistenbereichen dieser Personen führen.

Der Erfindung liegt die Aufgabe zugrunde, einen Leibgurt zu schaffen, bei dem eine großflächige Abstützung des Bauches beim Gehen und Stehen sichergestellt ist, ohne daß diese großflächige Abstützung mit Nachteilen beim Sitzen erkauft werden muß. Diese Aufgabe wird bei dem erfindungsgemäßen Leibgurt dadurch gelöst, daß mindestens zwei Stützgurte vorgesehen sind, daß die Stützgurte lediglich längselastisch sind und daß die oberen und unteren Ränder jeweils aufeinanderfolgender Stützgurte über jeweils eine längs- und querelastische Stoffbrücke miteinander verbunden sind, wobei jede Stoffbrücke beim Sichsetzen der den Leibgurt tragenden Person ein Übereinanderschieben der Stützgurte ermöglicht, die beim Aufstehen wieder in ihre Ausgangslage zurückbewegt werden können.

Der erfindungsgemäße Leibgurt bietet den Vorteil, daß er beim Sichsetzen der ihn tragenden Person um ein Maß schmaler wird, das lokale Druckbelastungen, wie sie bei einem einteiligen Gurt gleicher wirksamer Breite auftreten würden, ausschließt. Die die jeweils oberen und unteren Ränder aufeinanderfolgender Stützgurte miteinander verbindende elastische Stützbrücke sorgt beim Aufstehen der den Leibgurt tragenden Person dafür, daß die zusammengeschobenen Stützgurte nach Art eines sich öffnenden Visiers wieder in ihre Ausgangslage zurückbewegt werden, in der sie den Unterbauch großflächig abstützen und in der folglich die Gefahr des von schmalen Leibgurten bekannten sogenannten "Einschneidens" in das Bauchgewebe nicht gegeben ist.

Weitere Einzelheiten und Merkmale der Erfindung ergeben sich aus den Unteransprüchen und der nachstehenden Beschreibung zweier in der beigefügten Zeichnung dargestellter Ausführungsformen. Es zeigen:
Fig. 1 die perspektivische Ansicht eines Leibgurtes,
Fig. 2 schematisch die Lage der Stützgurte des Leibgurtes gemäß Fig. 1 in der Sitzposition eines Leibgurtträgers,
Fig. 3 schematisch die Lage der Stützgurte des Leibgurtes gemäß Fig. 1 bei einem stehenden Leibgurtträger,
Fig. 4 die Verwendung eines Leibgurtes gemäß Fig. 1 in Verbindung mit einer Miederhose und
Fig. 5 eine modifizierte Ausführungsform des Leibgurtes gemäß Fig. 1 bis 3.

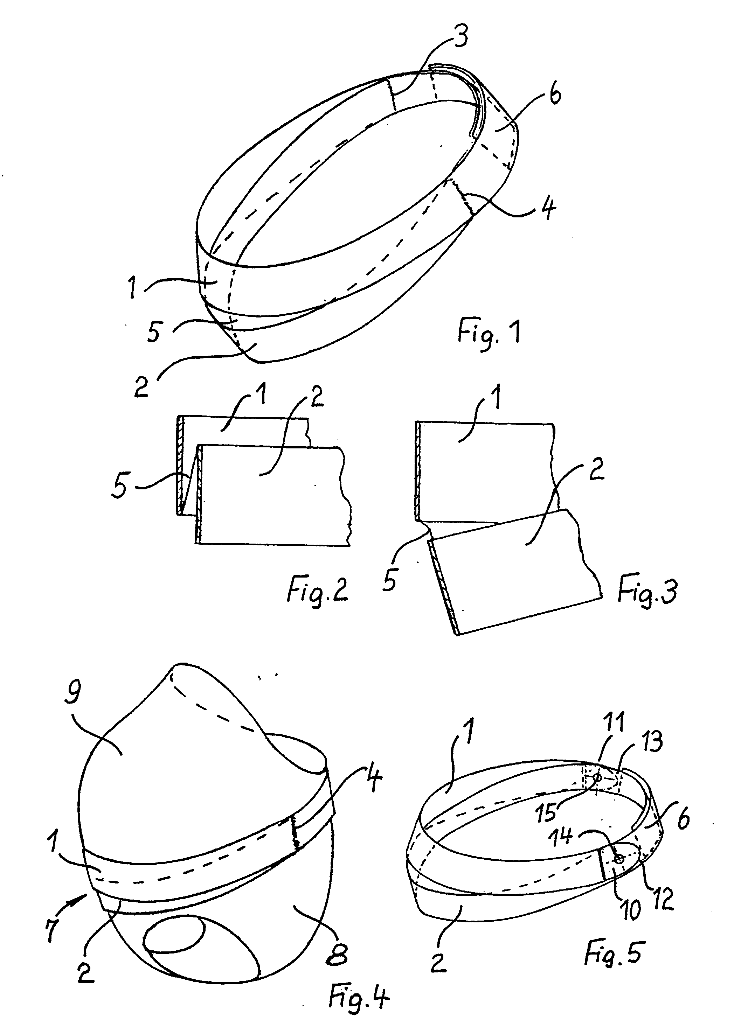

Der in den Figuren 1 bis 3 dargestellte Leibgurt weist zwei Stützgurte 1 und 2 auf, die im durch Quernähte 3 und 4 definierten Rückenbereich unmittelbar miteinander verbunden sind, während sie im zur Abstützung des Unterbauches dienenden Vorderteil des Leibgurtes lediglich mittelbar über eine hochelastische Stoffbrücke 5 miteinander in Verbindung stehen. Die Stützgurte 1 und 2 bilden im Rückenbereich des Leibgurtes mit anderen Worten zweilagige Abschnitte des Leibgurtes. Um den Leibgurt bequem öffnen und schließen zu können, sind mit den vorgenannten doppellagigen Abschnitten die miteinander zusammenwirkenden Teile eines Haftreißverschlusses 6 verbunden, der auch eine Längenveränderung des Gurtes zuläßt. Setzt sich die den Leibgurt tragende Person, so schieben sich die Stützgurte 1 und 2, wie in Figur 2 angedeutet, schuppenartig zusammen und zwar unter elastischer Dehnung der Stoffbrücke 5. Der Leibgurt wird mit anderen Worten schmaler. Der vom Leibgurt auf den Leistenbereich ausgeübte Druck bleibt dadurch vernachlässigt bzw. zumindest in vertretbaren Grenzen. Beim erneuten Aufstehen gehen die Stützgurte 1 und 2 - nicht zuletzt unter der Einwirkung der elastischen Stoffbrücke 5 - in ihre in Figur 3 angedeutete Ausgangsstellung zurück. Der Leibgurt paßt sich folglich an die unterschiedlichen, beim Sitzen und Stehen bzw. Gehen an ihn zu stellenden Anforderungen an.

Figur 4 zeigt die Möglichkeit der Integration eines Leibgurtes gemäß Fig. 1 bis 3 in eine Miederhose für werdende Mütter. Der Leibgurt 7 verbindet in diesem Fall einen Hosenteil 8 mit einem zur ergänzenden Abstützung des Oberbauches dienenden Leibteil 9. Selbstverständlich ist es auch möglich, einen Leibgurt nur mit einem Leibteil 9 oder einem Hosenteil 8 zu kombinieren.

In Figur 5 ist eine leicht modifizierte Ausführungsform des Leibgurtes gemäß Fig. 1 bis 3 dargestellt, bei der die Enden des Stützgurtes 1 mit Laschen 10 und 11 versehen sind, die ebenso wie die ihnen zugeordneten Abschnitte 12 und 13 des Stützgurtes 2 durch Stoff, Kunststoff oder Leder verstärkt sind. Die verstärkten Laschen 10 und 11 sind in diesem Fall über Scharniere 14,15 mit verstärkten Abschnitten 12,13 verbunden. Der modifizierte Leibgurt ist im Rückenbereich folglich nur einlagig ausgebildet.

## Patentansprüche

1. Leibgurt, der zwei sich im Bauchbereich mehr oder weniger schuppenartig überlappende elastische Stützgurte (1,2) aufweist, **dadurch gekennzeichnet,** daß mindestens zwei Stützgurte (1,2) vorgesehen sind, daß die Stützgurte (1,2) lediglich längselastisch sind und daß die oberen und unteren Ränder jeweils aufeinanderfolgender Stützgurte (1,2) über jeweils eine längs- und querelastische Stoffbrücke (5) miteinander verbunden sind, wobei jede Stoffbrücke (5) beim Sichsetzen der den Leibgurt tragenden Person ein Übereinanderschieben der Stützgurte (1,2) ermöglicht, die beim Aufstehen wieder in ihre Ausgangslage zurückbewegt werden können.

2. Leibgurt nach Anspruch 1, **dadurch gekennzeichnet,** daß die Enden mindestens eines Stützgurtes (1) durch Längsund Quernähte mit längs- und quersteifen Abschnitten eines Haftreißverschlusses (6) verbunden sind.

3. Leibgurt nach Anspruch 2, **dadurch gekennzeichnet,** daß die sich über die gesamte Höhe der im Rückenbereich dekkungsgleich miteinander verbundenen Stützgurte (1,2) erstreckenden Abschnitte des Haftreißverschlusses (6) eine die Längenänderung des Leibgurtes ermöglichende Breite haben.

4. Leibgurt nach Anspruch 1 oder 2, **dadurch gekennzeichnet,** daß die Enden eines Stützgurtes (1) mit Laschen (10,11) versehen und mit dem anderen Stützgurt (2) über Scharniere (14,15) verbunden sind.

5. Leibgurt nach Anspruch 4, **dadurch gekennzeichnet,** daß die Stützgurte (1,2) im Bereich ihrer Verbindungsstellen mit Verstärkungen aus Stoff, Kunststoff oder Leder versehen sind.

6. Leibgurt nach einem oder mehreren der Ansprüche 1 bis 5, **dadurch gekennzeichnet,** daß sich an den oberen Rand des obersten Stützgurtes ein zur Oberbauchabstützung dienender hochelastischer Leibteil (9) anschließt.

7. Leibgurt nach einem oder mehreren der Ansprüche 1 bis 6, **dadurch gekennzeichnet,** daß er Teil einer Miederhose mit einem Leibteil (9) und einem Hosenteil (8) ist.

## Claims

1. An abdominal belt with two elastic supporting straps (1,2) which overlap in an imbricated manner to a greater or lesser extent in the abdominal area, characterized in that at least two supporting straps (1,2) are designed, that the supporting straps (1,2) are merely longitudinally elastic, and that the upper and lower borders of respectively successive supporting straps (1,2) are connected to one another via in each case a longitudinally and transversely elastic fabric bridge (5) with each fabric bridge (5) ensuring, when the individual wearing the abdominal belt sits down, that the supporting straps (1,2) are pushed one over the other and can be moved back into their initial position when the individual stands up.

2. The abdominal belt as claimed in claim 1, characterized in that the ends of at least one supporting strap (1) are connected by longitudinal and transverse seams to longitudinally and transversely stiff sections of the touch-and-close fastener (6).

3. The abdominal belt as claimed in claim 2, characterized in that those sections of the touch-and-close fastener (6) which extend over the entire height of the supporting straps (1,2), connected to one another in a congruent manner in the back area, are of a width which permits the length of the abdominal belt to be changed.

4. The abdominal belt as claimed in claim 1 or 2, characterized in that the ends of one supporting strap (1) are provided with tabs (10,11) and are connected to the other supporting strap (2) via hinges (14,15).

5. The abdominal belt as claimed in claim 4, characterized in that, in the region of their connecting locations, the supporting belts (1,2) are provided with reinforcements made of fabric, plastic or leather.

6. The abdominal belt as claimed in one or more of claims 1 to 5, characterized in that the upper border of the uppermost supporting strap is adjoined by a highly elastic abdominal part (9) which serves for upper-abdomen support.

7. The abdominal belt as claimed in one or more of claims 1 to 6, characterized in that it is part of a panty girdle with an abdominal part (9) and a panty part (8).

## Revendications

1. Ceinture abdominale qui comporte deux sangles élastiques de soutien (1, 2) se recouvrant plus ou moins à la manière d'écailles, caractérisée en ce qu'il est prévu au moins deux sangles de soutien (1, 2), en ce que les sangles de soutien (1, 2) sont élastiques uniquement longitudinalement, et en ce que les bords supérieurs et bords inférieurs de sangles de soutien (1, 2) se succédant, sont reliés entre eux respectivement par un pont de tissu (5) élastique longitudinalement et transversalement, chaque pont de tissu (5) permettant, lorsque la personne qui porte la ceinture abdominale s'assoit, une superposition des sangles de soutien (1, 2) qui peuvent à nouveau être déplacées dans leur position initiale, lorsque la personne se met debout.

2. Ceinture abdominale selon la revendication 1, caractérisée en ce que les extrémités d'au moins une sangle de soutien (1) sont reliées par des coutures longitudinales et des coutures transversales à des parties rigides longitudinalement et transversalement d'une fermeture à accrochage (6).

3. Ceinture abdominale selon la revendication 2, caractérisée en ce que les parties de la fermeture à accrochage (6) qui s'étendent sur toute la hauteur des sangles de soutien (1, 2), reliées entre elles de manière à coïncider dans la région du dos, ont une largeur qui permet de faire varier la longueur de la ceinture abdominale.

4. Ceinture abdominale selon la revendication 1 ou 2, caractérisée en ce que les extrémités d'une sangle de soutien (1) son pourvues de pattes (10, 11) et sont reliées à l'autre sangle de soutien (2) par des charnières (14, 15).

5. Ceinture abdominale selon la revendication 4, caractérisée en ce que les sangles de soutien (1, 2) sont pourvues de renforts en tissu, matière plastique ou cuir, dans la région de leurs points d'assemblage.

6. Ceinture abdominale selon une ou plusieurs des revendications 1 à 5, caractérisée en ce qu'un corset (9) très élastique, et qui sert à soutenir la partie supérieure du ventre, se raccorde au bord supérieur de la sangle de soutien la plus haute.

7. Ceinture abdominale selon une ou plusieurs des revendications 1 à 6, caractérisée en ce qu'elle fait partie d'une gaine-culotte avec un corset (9) et une culotte (8) .
